(19)

Europäisches Patentamt
European Patent Office
Office européen des brevets

(11) **EP 4 513 496 A1**

(12) **EUROPEAN PATENT APPLICATION**

(43) Date of publication:
26.02.2025 Bulletin 2025/09

(21) Application number: 23213715.8

(22) Date of filing: 01.12.2023

(51) International Patent Classification (IPC):
G16B 20/00 (2019.01)    C12Q 1/6886 (2018.01)
G16B 20/20 (2019.01)    G16B 30/00 (2019.01)
G16H 50/20 (2018.01)

(52) Cooperative Patent Classification (CPC):
G16B 20/20; G16B 30/00; G16H 50/20;
C12Q 1/6886

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 22.08.2023 KR 20230109872

(71) Applicant: Inocras Korea Inc.
Daejeon 34051 (KR)

(72) Inventors:
• Ju, Young Seok
34051 DAEJEON (KR)
• Lee, Won-Chul
34051 DAEJEON (KR)

(74) Representative: Glawe, Delfs, Moll
Partnerschaft mbB von
Patent- und Rechtsanwälten
Postfach 13 03 91
20103 Hamburg (DE)

Remarks:
Amended claims in accordance with Rule 137(2)
EPC.

(54) **METHOD AND APPARATUS FOR DETECTING MINIMAL RESIDUAL DISEASE USING TUMOR INFORMATION**

(57) A method for detecting minimal residual disease using tumor information is provided, including acquiring first sequencing data associated with a first sample from a patient, acquiring second sequencing data associated with a second sample from the patient, acquiring third sequencing data associated with a third sample from the patient, and performing detection of minimal residual disease for the patient based on the first sequencing data, the second sequencing data, and the third sequencing data.

FIG. 1

**Description**

## BACKGROUND

TECHNICAL FIELD

The present disclosure relates to a method and apparatus for detecting minimal residual disease using tumor information, and more specifically, to a method and apparatus for detecting minimal residual disease in a patient using mutation information from tumor tissue and liquid biopsy samples.

RELATED ART

Genetic analysis technology is widely utilized in the medical field to identify genes of a living organism and determine its characteristics or traits, etc. In recent years, medical practice for understanding and treating diseases such as tumors has evolved from a traditional prescription-centered approach to precision medicine. This is form of treatment that considers the genetic information, health records and other factors specific to individual patients.

In the field of precision medicine, it is crucial to collect extensive personal genetic information and perform clinical analysis accordingly. In recent years, Next-Generation Sequencing (NGS) technology, which can rapidly read large amounts of DNA information in parallel, has found widespread use in various medical applications, including cancer screening. The NGS technology offers the advantages of convenience, reduced cost and shorter genome analysis time. However, it does come with the inherent limitation of generating a background error rate ranging from approximately 0.1% to 1%. In other words, even for DNA without mutations, there is a statistical limitation where one base pair out of every 100 to 1,000 base pairs may be incorrectly identified as having a mutation (i.e., an error rate of 0.1% to 1%) during the sequencing process.

Due to these inherent limitations of NGS technology, it faces challenges in detecting DNA mutations present at very low rates. For example, cancer-derived DNA (e.g., ctDNA: Circulating tumor DNA) generally exists in concentrations of less than 1% in the blood of cancer patients. Therefore, it is challenging to distinguish whether the mutations detected in the blood using the NGS technology originate from cancer tissue or are misidentified due to the background error rate. Therefore, there is a need for a new technology that can detect DNA mutations (such as tumor cell remnants) present at very low rates in the patient's blood while reducing the background error rate.

## SUMMARY

In order to address one or more problems (e.g., the problems described above and/or other problems not explicitly described herein), the present disclosure provides a method for, a non-transitory computer-readable recording medium storing instructions for, and an apparatus (system) for detecting minimal residual disease using tumor information.

The present disclosure may be implemented in a variety of ways, including a method, a system (apparatus), or a computer program stored in a readable storage medium.

A method for detecting minimal residual disease is provided, which may be performed by one or more processors and include acquiring first sequencing data associated with a first sample from a patient, acquiring second sequencing data associated with a second sample from the patient, acquiring third sequencing data associated with a third sample from the patient, and performing detection of minimal residual disease for the patient based on the first sequencing data, the second sequencing data, and the third sequencing data.

The first sequencing data, the second sequencing data, and the third sequencing data may be acquired through whole genome sequencing (WGS).

The first sample may be a tumor tissue biopsy sample of the patient, the second sample may be a normal blood sample of the patient, and the third sample may be a plasma sample of the patient, and the plasma sample may include cell free Deoxyribo Nucleic Acid (cfDNA) and circulating tumor Deoxyribo Nucleic Acid (ctDNA).

The first and second samples may be samples acquired at a first time point, the third sample may be a sample acquired at a second time point after the first time point, and at least one of surgery or therapy may be performed on the patient after the first time point and before the second time point.

The method may further include detecting tumor tissue mutation information of the patient by comparing the first sequencing data and the second sequencing data, and performing background error filtering on the detected tumor tissue mutation information using genetic data associated with a plurality of sample patients that are distinguished from the patient.

The genetic data may include sequencing data for normal blood samples of the plurality of sample patients, and the performing the filtering may include removing, mutations among the detected tumor tissue mutations, which are detected in sequencing data for the normal blood samples of the plurality of sample patients from the tumor tissue mutation information.

The genetic data may include sequencing data for plasma samples from the plurality of sample patients, and the performing the filtering may include removing, of the detected tumor tissue mutations, mutations detected in sequencing data for the plasma samples from the plurality of sample patients from the tumor tissue mutation information.

The performing the detection of minimal residual disease may include calculating a limit of detection for the patient based on the first sequencing data, the second sequencing data, and the third sequencing data, calculating a tumor cell fraction (TCF) for the patient based on the third sequencing data, correcting the tumor cell fraction using genetic data associated with a plurality of sample patients distinguished from the patient, and determining whether the patient has tumor recurrence based on the corrected tumor cell fraction and the limit of detection.

The limit of detection may represent the lowest tumor cell fraction that can be detected in the plasma sample of the patient.

The limit of detection may be calculated based on the number of tumor tissue mutations of the patient detected by comparing the first sequencing data and the second sequencing data and an average sequencing depth of the third sequencing data.

The calculating the tumor cell fraction may include determining the number of reads that are different from a reference sequence in the third sequencing data, determining the number of reads that match the reference sequence in the third sequencing data, and calculating the tumor cell fraction based on the number of reads that are different from the reference sequence and the number of reads that match the reference sequence.

The determining the number of reads that are different from a reference sequence may include detecting tumor tissue mutation information of the patient by comparing the first sequencing data and the second sequencing data, determining the number of reads in the third sequencing data including mutations detected in tumor tissue based on the tumor tissue mutation information, and using the number of reads in the third sequencing data including the mutations detected in the tumor tissue as the number of reads that are different from the reference sequence.

The correcting the tumor cell fraction may include determining the number of reads that are different from a reference sequence in plasma sequencing data included in genetic data associated with a plurality of sample patients distinguished from the patient, determining the number of reads that match the reference sequence in the plasma sequencing data included in the genetic data associated with the plurality of sample patients distinguished from the patient, calculating a random error rate based on the number of reads that are different from the reference sequence and the number of reads that match the reference sequence, and correcting the tumor cell fraction using the random error rate.

The determining whether the patient has tumor recurrence may include calculating a confidence interval of a predetermined confidence level of the corrected tumor cell fraction, and in response to determining that a lower limit of the calculated confidence interval is higher than the calculated limit of detection, determining that the patient's tumor has recurred.

The performing the detection of minimal residual disease may further include generating a first chromosomal arm-level copy number profile based on the first sequencing data, generating a second chromosomal arm-level copy number profile arm based on the second sequencing data, generating a third chromosomal arm-level copy number profile based on the third sequencing data, and calculating a tumor cell fraction based on the first to third chromosomal arm-level copy number profiles.

The performing the detection of minimal residual disease may further include identifying a first set of structural variants detected from the first sequencing data, identifying a second set of structural variants detected from the third sequencing data, and determining whether the patient has tumor recurrence based on a comparison result of the first set of structural variants and the second set of structural variants.

The determining whether the patient has tumor recurrence may include determining that the tumor has recurred in the patient if the number or ratio of structural variants included in the second set of structural variants among the first set of structural variants is equal to or greater than a threshold.

The first set of structural variants may include at least one of inversion, translocation, duplication, deletion, or insertion in some region of the genome.

A computer-readable non-transitory recording medium storing instructions for executing the method for detecting minimal residual disease using tumor information on a computer is provided.

A system is provided, which may include a communication module, a memory, and one or more processors connected to the memory and configured to execute one or more computer-readable programs included in the memory, in which the one or more programs may include instructions for acquiring first sequencing data associated with a first sample from a patient, acquiring second sequencing data associated with a second sample from the patient, acquiring third sequencing data associated with a third sample from the patient, and performing detection of minimal residual disease for the patient based on the first sequencing data, the second sequencing data, and the third sequencing data.

## TECHNICAL IMPROVEMENT

According to various examples of the present disclosure, by reducing the background error rate through data filtering and lowering the limit of detection of minimal residual disease (MRD) in non-invasive liquid biopsy samples, tumor cells can be detected earlier.

According to various examples of the present disclosure, by calculating the tumor cell fraction based on the filtered tumor tissue mutations, the time and computing resources required to compare whole plasma sequencing data to the reference sequences can be reduced.

According to various examples of the present disclosure, by filtering tumor tissue mutations of the target patient using the genetic data of the sample patient, the tumor cell fraction in the target patient's body can be predicted very accurately.

According to various examples of the present disclosure, by using the chromosomal arm-level copy number profile of the target patient, the tumor cell fraction in the target patient's body can be predicted very accurately.

The effects of the present disclosure are not limited to the effects described above, and other effects not mentioned will be able to be clearly understood by those of ordinary skill in the art (referred to as "those skilled in the art") from the description of the claims.

## BRIEF DESCRIPTION OF THE DRAWINGS

The above and other objects, features and advantages of the present disclosure will be described with reference to the accompanying drawings described below, where similar reference numerals indicate similar elements, but not limited thereto, in which:

FIG. 1 is a diagram illustrating an example of a process for detecting minimal residual disease in a target patient;
FIG. 2 is a schematic diagram illustrating a configuration in which an information processing system is communicatively connected to a plurality of user terminals to provide a service for detecting minimal residual disease using tumor information;
FIG. 3 is a block diagram illustrating an internal configuration of the user terminal and the information processing system;
FIG. 4 is a diagram illustrating an example of a graph depicting the tumor cell fraction in the body of a target patient over time;
FIG. 5 is a diagram illustrating a blood sample from a target patient;
FIG. 6 is a diagram illustrating a process of calculating a limit of detection for a target patient;
FIG. 7 is a diagram illustrating a process of tumor detection;
FIG. 8 is a diagram illustrating example graphs illustrating the relationship between the number of mutations detected in tumor cells of a target patient and the limit of detection of the target patient;
FIG. 9 is a diagram illustrating an example of generating an arm-level copy number profile;
FIG. 10 is a diagram illustrating an example of calculating the tumor cell fraction in the body of a target patient using a chromosomal arm-level copy number profile;
FIG. 11 is a diagram illustrating performance verification results of a method for detecting minimal residual disease in a target patient using genetic data from a sample patient;
FIG. 12 is a diagram illustrating performance verification results of a method for detecting minimal residual disease in a target patient using a chromosomal arm-level copy number profile of the target patient; and
FIG. 13 is a flowchart illustrating a method for detecting minimal residual disease using tumor information.

## DETAILED DESCRIPTION

Hereinafter, example details for the practice of the present disclosure will be described in detail with reference to the accompanying drawings. However, in the following description, detailed descriptions of well-known functions or configurations will be omitted if it may make the subject matter of the present disclosure rather unclear.

In the accompanying drawings, the same or corresponding components are assigned the same reference numerals. In addition, in the following description of various examples, duplicate descriptions of the same or corresponding components may be omitted. However, even if descriptions of components are omitted, it is not intended that such components are not included in any example.

Advantages and features of the disclosed examples and methods of accomplishing the same will be apparent by referring to examples described below in connection with the accompanying drawings. However, the present disclosure is not limited to the examples disclosed below, and may be implemented in various forms different from each other, and the examples are merely provided to make the present disclosure complete, and to fully disclose the scope of the disclosure to those skilled in the art to which the present disclosure pertains.

The terms used herein will be briefly described prior to describing the disclosed example(s) in detail. The terms used herein have been selected as general terms which are widely used at present in consideration of the functions of the present disclosure, and this may be altered according to the intent of an operator skilled in the art, related practice, or introduction of new technology. In addition, in specific cases, certain terms may be arbitrarily selected by the applicant, and the meaning of the terms will be described in detail in a corresponding description of the example(s). Therefore, the terms used in the present disclosure should be defined based on the meaning of the terms and the overall content of the present disclosure rather than a simple name of each of the terms.

As used herein, the singular forms "a," "an," and "the" are intended to include the plural forms as well, unless the context clearly indicates the singular forms. Further, the plural forms are intended to include the singular forms as well, unless the context clearly indicates the plural forms. Further, throughout the description, when a portion is stated as "comprising (including)" a component, it is intended as meaning that the portion may additionally comprise (or include or have) another component, rather than excluding the same, unless specified to the contrary.

Further, the term "module" or "unit" used herein refers to a software or hardware component, and "module" or "unit" performs certain roles. However, the meaning of the "module" or "unit" is not limited to software or hardware. The "module" or "unit" may be configured to be in an addressable storage medium or configured to play one or more processors. Accordingly, as an example, the "module" or "unit" may include components such as software components, object-oriented software components, class components, and task components, and at least one of processes, functions, attributes, procedures, subroutines, program code segments, drivers, firmware, micro-codes, circuits, data, database, data structures, tables, arrays, and variables. Furthermore, functions provided in the components and the "modules" or "units" may be combined into a smaller number of components and "modules" or "units", or further divided into additional components and "modules" or "units."

The "module" or "unit" may be implemented as a processor and a memory. The "processor" should be interpreted broadly to encompass a general-purpose processor, a central processing unit (CPU), a microprocessor, a digital signal processor (DSP), a controller, a microcontroller, a state machine, etc. Under some circumstances, the "processor" may refer to an application-specific integrated circuit (ASIC), a programmable logic device (PLD), a field-programmable gate array (FPGA), etc. The "processor" may refer to a combination of processing devices, e.g., a combination of a DSP and a microprocessor, a combination of a plurality of microprocessors, a combination of one or more microprocessors in conjunction with a DSP core, or any other combination of such configurations. In addition, the "memory" should be interpreted broadly to encompass any electronic component that is capable of storing electronic information. The "memory" may refer to various types of processor-readable media such as random access memory (RAM), read-only memory (ROM), non-volatile random access memory (NVRAM), programmable read-only memory (PROM), erasable programmable read-only memory (EPROM), electrically erasable PROM (EEPROM), flash memory, magnetic or optical data storage, registers, etc. The memory is said to be in electronic communication with a processor if the processor can read information from and/or write information to the memory. The memory integrated with the processor is in electronic communication with the processor.

In this disclosure, "whole genome sequencing (WGS)" may refer to a technology used for determining the complete DNA sequence of an organism's genome. Specifically, WGS may involve deciphering and identifying the order of nucleotide bases (adenine, cytosine, guanine, and thymine) within the whole set of genetic material of a person or organism. The whole set of genetic material may include all genes, non-coding regions, and any additional genetic elements present in the genome. WGS typically proceeds through several steps, including DNA extraction from specific cells, fragmentation of the extracted DNA into smaller fragments, and generation of millions or billions of short DNA sequences called "reads." These generated reads may be then aligned and assembled to reconstruct the whole genome sequence. In certain instances, various duplex sequencing methods, such as the Concatenating Original Duplex for Error Correction (CODEC) method which is an example of duplex sequencing, may be employed to minimize the background error rate, allowing even the smallest cancer-derived DNA to be detected from blood. In this regard, PCT Publication WO 2022/125997 A1, published on June 16, 2022, is incorporated herein by reference.

In the present disclosure, "DNA" encompasses not only DNA itself, but also any nucleic acid or nucleic acid sequence, such as Ribonucleic Acid (RNA).

In the present disclosure, "cfDNA" may refer to circulating free DNA, also called cell free DNA or circulating DNA. cfDNA may refer to small fragments of DNA released into the bloodstream and/or other body fluids by cells undergoing normal cell death or as a result of certain pathological processes. cfDNA may consist of short DNA fragments originating from various tissues in the body, including both healthy and diseased cells. cfDNA may originate from organs such as the liver, heart, or lungs, and also from tumors (e.g., malignant tumors such as cancer). For example, by centrifuging a blood sample, it is possible to obtain the plasma layer, buffy coat layer, and red blood cell layer in order from the upper layer, and cfDNA may be obtained from the plasma layer of these layers.

In the present disclosure, "ctDNA" may refer to circulating tumor DNA. ctDNA may be a subset of cfDNA specifically originating from tumor cells. Tumor cells may release ctDNA into the bloodstream as they undergo apoptosis or cell death. ctDNA may exhibit genetic alterations or mutations that are characteristic of the tumor from which they originate. Analyzing ctDNA may provide valuable insights into the genetic characteristics and mutational profile of tumors without the need for invasive tissue biopsies. By analyzing ctDNA, it is possible to monitor a patient's response to treatment and detect minimal residual disease (MRD).

In the present disclosure, the minimal residual disease (MRD) can be small number of cancer cells or tumor cells, which remain in the body after treatment. The number of remaining cells may be so small that they do not cause any physical signs or symptoms and their detection often poses a significant challenge. Traditional methods, such as monitoring abnormal serum proteins in the blood provide some insight, but may not always detect these minute quantities of residual cells. In the realm of imaging, techniques such as Computed Tomography (CT) and Magnetic Resonance Imaging (MRI) are utilized. CT scans can identify subtle lesions or tissue density changes, while MRI offers detailed images of soft tissues, potentially indicating the presence of MRD. While these methods contribute to the detection process, they have limitations in sensitivity and specificity, leading to the exploration of more advanced techniques. The detection of the MRD means that residual (remaining) disease was detected.

In the present disclosure, "normal blood sample" or "buffy coat" may refer to specific components of a blood sample including white blood cells and platelets. These samples can be obtained by centrifuging the blood sample to create a thin layer between the red blood cell layer at the bottom and the plasma layer at the top. The normal blood samples may serve as a source of genomic DNA derived from an individual's cells, including higher concentrations of nucleated cells, such as white blood cells with nuclei containing DNA. During WGS, DNA can be extracted from the normal blood sample, providing a specific individual's genomic DNA that can serve as reference or control DNA. This enables the comparisons and identification of genetic mutations or mutations present in cfDNA or ctDNA extracted from the same individual, as well as DNA from tumor tissue extracted from the same individual . It is typically assumed that the normal blood sample does not contain cancer cells.

In the present disclosure, "sequencing depth" or "sequencing coverage" may refer to the average number of times each base of the genome is read during the sequencing process. For example, the sequencing depth may indicate how many times a specific nucleotide (A, T, C, or G) at a particular location in the genome is sequenced. The sequencing depth is a parameter affecting the accuracy and reliability of the sequencing data. The sequencing depth is often measured in units of "X", where 1X coverage means each base is sequenced once on average, and 10X coverage means each base is sequenced 10 times on average.

In the present disclosure, "copy number profiling" may refer to a technology for acquiring a copy number profile through the analysis of copy number variations in specific DNA segments or regions within the genome using WGS data.

The copy number profiling may include several steps. First, a DNA isolation step for isolating DNA from the cell or tissue of interest may be performed. Subsequently, a sequencing step for dividing the extracted DNA into smaller fragments (fragmentation) and performing sequencing on the DNA fragments may be performed. With this step, it is possible to generate vast amounts of reads, which are short DNA sequences. In some embodiments, after dividing the DNA into fragments and before performing the sequencing step, a step of amplifying and cloning the DNA fragments using a technique such as polymerase chain reaction (PCR) may be performed.

A read alignment step in which the generated reads are aligned or mapped to a reference sequence may be performed. This step may involve comparing the read to a known DNA sequence of a reference sequence to determine the original location of the reads. Once the reads are aligned, software tools may be used to perform a copy number analysis step of analyzing the sequencing depth or sequencing coverage of the whole genome. The sequencing coverage may indicate the number of reads aligned to a specific genomic region. A normalization step may be performed on the coverage data to account for changes in the sequencing depth and other technical biases. This allows accurate comparisons between different genomic regions.

A copy number calling step involves applying a statistical algorithm to the normalized coverage data to identify genomic regions exhibiting copy number mutations. This algorithm may compare observed coverage to expected coverage based on a diploid genome. In some embodiments, this algorithm may be autonomously performed by a hardware device to provide a prompt identification result. The resulting copy number profile may be visualized using plots or heatmaps, showing genomic regions with copy number gains or losses. This step may be referred to as the "visualization and interpretation step," and the visualization data may provide insight into genomic alterations like amplifications, deletions, or duplications. In some embodiments, a hardware device autonomously generates a visualization data by using an artificial intelligence (AI)-based tool for generating graphs.

In the present disclosure, a "limit of detection" may refer to a minimum detection concentration that can confirm the presence or absence of an analyte material in the analysis process for the analyte material. For example, the "limit of detection" may refer to the lowest tumor cell fraction that can be detected in a plasma sample from a tumor patient.

In the present disclosure, the term "sample patient" may refer to any individual or any subject from whom tissue samples, blood samples, and/or genetic data, including sequencing data, or similar information is collected. It is not necessarily limited to patients, in the medical sense, who are under or require medical care.

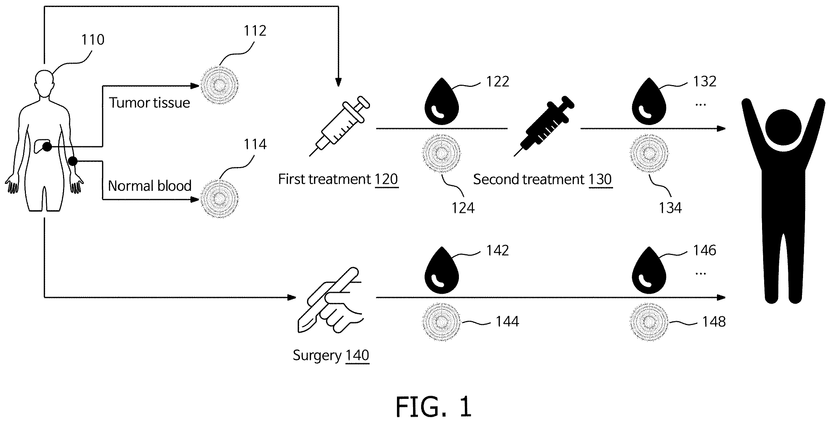

FIG. 1 is a diagram illustrating an example of a process for detecting minimal residual disease in a target patient 110, who may be a patient with tumor tissue (e.g., cancer tissue). In addition, the target patient 110 may be a subject for whom detection of minimal residual disease is performed after treatment 120 and 130 or surgery 140 on tumor tissue is performed.

At a first time point before undergoing the treatment 120 and 130 or the surgery 140 on tumor tissue, a tumor tissue biopsy sample and a normal blood sample may be collected from the target patient 110. The normal blood sample may be acquired by centrifuging blood collected from the target patient 110. Tumor tissue sequencing data 112 and normal blood sequencing data 114 may be generated based on these samples, using whole genome sequencing (WGS).

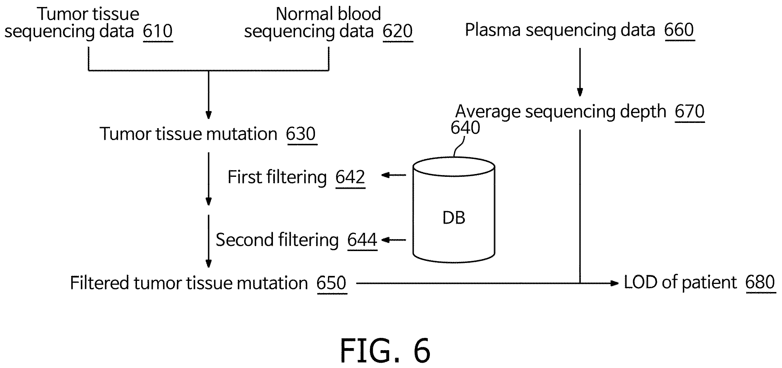

Tumor tissue mutation information of the target patient 110 may then be detected based on the acquired tumor tissue sequencing data 112 and normal blood sequencing data 114. Background error filtering may be applied to this information using genetic data from a variety of sample patients distinct from the target patient 110. As a result of this background error filtering, the background error rate is reduced, enabling earlier confirmation of tumor recurrence in the target patient 110. That is, the background error filtering may achieve a technical improvement that the background error rate is reduced, and that the accuracy of the analysis is increased. Further details regarding the process of detecting and filtering tumor tissue mutation information will be described below with reference to FIG. 6.

Following the collection of the tumor tissue biopsy sample and the normal blood sample from the target patient 110, the target patient 110 may undergo at least one of treatments 120 and 130 and/or the surgery 140. As these interventions are carried out, the tumor cell fraction in the body of the target patient 110 may decrease. However, over time, the tumor cell fraction in the body of the target patient 110 may increase again.

In order to quickly respond to disease recurrence resulting from an increase in tumor cells in the body, it is important to detect an increase in the tumor cell fraction early. To this end, a plasma sample from the target patient 110 may be collected after the treatment 120 and 130 and/or the surgery 140 is performed. The plasma sample may be obtained by centrifuging blood 122, 132, 142, and 146 collected from the target patient 110. The plasma sample may contain cell free Deoxyribo Nucleic Acid (cfDNA) and circulating tumor Deoxyribo Nucleic Acid (ctDNA). The plasma sample may be collected from the target patient 110 and analyzed at regular intervals or as needed so as to be used for follow-up tests for tumor recurrence. Specifically, plasma sample sequencing data 124, 134, 144, and 148 may be generated based on the plasma samples contained in the blood 122, 132, 142, and 146. The acquired plasma sample sequencing data 124, 134, 144, and 148 may be used to determine whether additional treatment and/or surgery is required for the target patient 110.

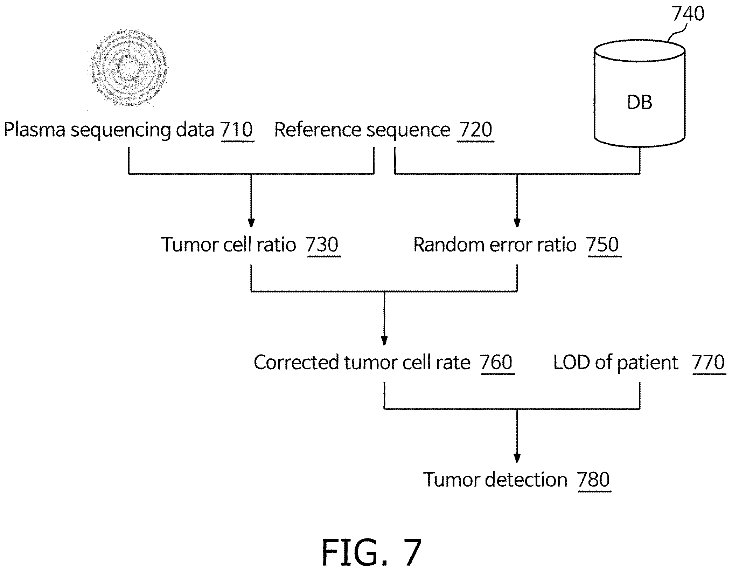

For example, the plasma sample sequencing data 124 may be obtained from the blood 122 collected at a second time point following the first treatment 120. The limit of detection for the tumor tissue of the target patient 110 may be calculated based on the tumor tissue mutation information and the plasma sample sequencing data 124, allowing for the detection of minimal residual disease in the target patient 110. The process of calculating the limit of detection of the tumor tissue of the target patient 110 will be described below in detail with reference to FIG. 6. In addition, the process of detecting minimal residual disease in the target patient 110 will be described below using FIG. 7.

If minimal residual disease is detected in the target patient 110 after the first treatment 120 (or if it is determined that tumor recurrence has occurred), the second treatment 130 may be performed. At a third time point after the second treatment 130, the blood 132 may be collected from the target patient 110, and the process of detecting minimal residual disease, as described above, may be repeated.

In another example, the minimal residual disease may not be detected based on the plasma sample sequencing data 144 acquired from the blood 142 collected at a fourth time point after the surgery 140. In this case, it is determined that the tumor has not recurred in the target patient 110, and no additional treatment and/or surgery is required. The process of detecting minimal residual disease may be repeated based on the plasma sample sequencing data 148 obtained from the blood 146 collected at a fifth time point after the fourth time point.

In summary, the blood of the target patient 110, who has undergone the treatment 120 and 130 and/or the surgery 140, may be collected at predetermined intervals or as needed. The plasma sample sequencing data 124, 134, 144, and 148 may be generated based on the plasma samples extracted from the collected blood. The detection of minimal residual disease may be performed based on the plasma sample sequencing data 124, 134, 144, and 148. If minimal residual disease is detected, additional treatment and/or surgery may be conducted for the target patient 110. By this way, outcomes from minimal residual disease detection can significantly refine and improve treatment strategies.

For example, in a treatment for a patient with breast cancer, if the minimal residual disease detection reveals a high tumor cell fraction (i.e., a tumor cell fraction which is higher than a predetermined threshold level), with a specific genetic mutation (e.g., HER2-positive), the treatment might shift from a standard chemotherapy to a targeted therapy like Trastuzumab or Pertuzumab. Conversely, a low minimal residual disease level (e.g., a tumor cell fraction which is lower than a predetermined threshold level) might indicate the effectiveness of the current treatment, leading to its continuation or a reduction in treatment intensity. In some embodiments, these processes can be performed by a hardware processor autonomously. In some embodiments, a hardware processor may determine whether to shift from a standard chemotherapy to a targeted therapy based on the received result of the minimal residual disease detection.

For another example, in a treatment for a patient with colon cancer, the presence of a high tumor cell fraction in minimal residual disease detection with specific mutations (e.g., KRAS, NRAS, or BRAF) might necessitate a change from standard chemotherapy to targeted agents like Cetuximab or Panitumumab, or in the case of BRAF mutations, to Vemurafenib. Lower levels of the minimal residual disease detection might suggest a reduction in treatment intensity or continuation with the current regimen. In some embodiments, these processes can be performed by a hardware processor autonomously. In some embodiments, a hardware processor may determine whether to change from a standard chemotherapy to targeted agents based on the received result of the minimal residual disease detection.

For another example, in a treatment for a patient with lung cancer, for non-small cell lung cancer (NSCLC) patients, a high minimal residual disease detection level with specific mutations (e.g., EGFR, ALK, ROS1) might lead to the initiation of targeted therapies such as Erlotinib, Crizotinib, or Lorlatinib. Lower minimal residual disease detection levels might indicate the effectiveness of current treatments or the possibility of reducing treatment intensity. In some embodiments, these processes can be performed by a hardware processor autonomously. In some embodiments, a hardware processor may determine whether to lead to the initiation of targeted therapies based on the received result of the minimal residual disease detection.

For another example, in a treatment for a patient with Hepatocellular Carcinoma (HCC), in the case of liver cancer, if the minimal residual disease detection reveals a high tumor cell fraction, especially with specific mutations like AFP (alpha-fetoprotein) elevations or mutations in genes such as TP53 or CTNNB1 (β-catenin), the treatment strategy might shift from standard therapies to targeted therapies like Sorafenib or Lenvatinib, which are specifically used in advanced HCC. Alternatively, if the minimal residual disease detection levels are low, it might suggest the efficacy of the current treatment, potentially leading to its continuation or a reduction in treatment intensity. In some cases, immunotherapies like Nivolumab or Pembrolizumab may be considered, especially in advanced stages or if the tumor shows certain immunogenic characteristics. In some embodiments, these processes can be performed by a hardware processor autonomously. In some embodiments, a hardware processor may determine whether to shift from a standard therapies to targeted therapies based on the received result of the minimal residual disease detection.

In each scenario, the minimal residual disease detection and its level can directly determine the course of treatment, allowing for a more tailored and potentially more effective approach to cancer therapy by using a medical device.

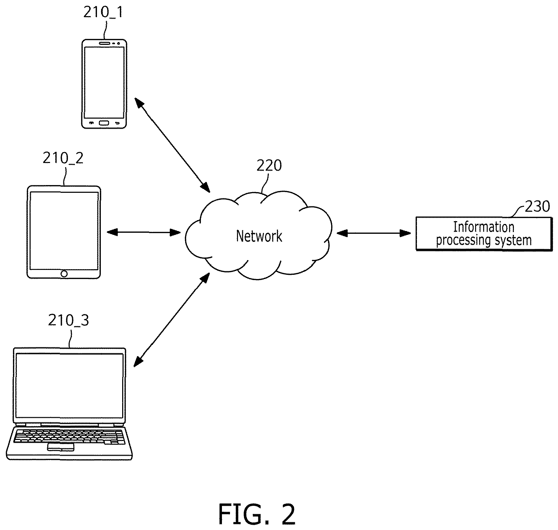

FIG. 2 is a schematic diagram illustrating a configuration in which an information processing system 230 is communicatively connected to a plurality of user terminals 210_1, 210_2, and 210_3 to provide a service for detecting minimal residual disease using tumor information. The information processing system 230 may include system(s) that can provide a service for detecting minimal residual disease using tumor information. The information processing system 230 may include one or more server devices and/or databases, or one or more distributed computing devices and/or distributed databases based on cloud computing services. These components may be capable of storing, providing, and executing computer-executable programs (e.g., downloadable applications) and data associated with the service for detecting minimal residual disease using tumor information. For example, the information processing system 230 may include separate systems (e.g., servers) for the service for detecting minimal residual disease using tumor information.

The service for detecting minimal residual disease using tumor information and other related services, provided by the information processing system 230 may be accessible to the user through an application or other software installed on each of the plurality of user terminals 210_1, 210_2, and 210_3. For example, the plurality of user terminals 210_1, 210_2, and 210_3 may be user terminals of experts in a medical institution, user terminals of patients, and the like.

The plurality of user terminals 210_1, 210_2, and 210_3 may communicate with the information processing system 230 through the network 220. The network 220 may be configured to enable communication between the plurality of user terminals 210_1, 210_2, and 210_3 and the information processing system 230. The network 220 may be configured as a wired network such as Ethernet, a wired home network (Power Line Communication), a telephone line communication device and RS-serial communication, a wireless network such as a mobile communication network, a wireless LAN (WLAN), Wi-Fi, Bluetooth, and ZigBee, or a combination thereof, depending on the installation environment. The method of communication is not limited, and may include a communication method using a communication network (e.g., mobile communication network, wired Internet, wireless Internet, broadcasting network, satellite network, etc.) that may be included in the network 220 as well as short-range wireless communication between the user terminals 210_1, 210_2, and 210_3.

In FIG. 2, a mobile phone terminal 210_1, a tablet terminal 210_2, and a PC terminal 210_3 are illustrated as examples of the user terminals, but aspects are not limited thereto, and the user terminals 210_1, 210_2, and 210_3 may be any computing device capable of wired and/or wireless communication and on which an application, etc. can be installed and executed. For example, the user terminal may include a medical device, a smartphone, a mobile phone, a navigation system, a vehicle black box, a computer, a notebook computer, a digital broadcasting terminal, Personal Digital Assistants (PDA), a Portable Multimedia Player (PMP), a tablet PC, a game console, a wearable device, an internet of things (IoT) device, a virtual reality (VR) device, an augmented reality (AR) device, etc. In addition, FIG. 2 illustrates that three user terminals 210_1, 210_2, and 210_3 are in communication with the information processing system 230 through the network 220, but aspects are not limited thereto, and a different number of user terminals may be configured to be in communication with the information processing system 230 through the network 220.

In some embodiments, in response to a detection of the minimal residual disease, the information processing system 230 transmits a signal including information on a type of additional treatment and/or surgery, which should be conducted for the target patient, to one or more of user terminals 210_1, 210_2, and 210_3. In some embodiments, the information processing system 230 decides a type of additional treatment and/or surgery, to conducted for the target patient, based on a result of the detection of the minimal residual disease, as aforementioned in the examples related to the breat cancer, colon cancer, lung cancer, hepatocellular carcinoma, mentioned above, and the processing system 230 transmits a signal including information on the decided type of additional treatment and/or surgery, to one or more of user terminals 210_1, 210_2, and 210_3. In some embodiments, , the information processing system 230 decides a type of additional treatment and/or surgery, to conducted for the target patient, by using a machine learning process using a neural network model. The neutral network model can be implemented as collections of nodes (neurons) that are connected in an acyclic graph. One type of neural network, a "feedforward network", can receive an input (a single vector) at its input layer of nodes, and, through a series of hidden layers, map the input to values in an output layer of nodes. Each layer in the network is made up of a set of neurons, where each neuron is fully connected to all neurons in the adjacent layer(s), and where neurons within a layer do not share any connections. If the network is modeling a classification, each of the nodes in the output layer may represent one of the possible classes to which an entity belongs, and the value of each output node may represent the probability that the input entity belongs to that class. A convolutional neural network (CNN) is another type of neural network model that can model feature detection by performing convolution operations on input data. For example, in image processing, a CNN may receive raw image data input and then output a set of feature maps representing abstractions of the raw data.

In some embodiments, the user terminals 210_1, 210_2, and 210_3 may include a hardware terminal managed by a doctor, a hardware terminal managed by a hospital, a hardware terminal managed by an insurance company, and a hardware terminal managed by a patient. In some embodiments, one of more of the user terminals 210_1, 210_2, and 210_3 may receive a signal to display a notification regarding the type of additional treatment and/or surgery, which should be conducted for the target patient.

In some embodiments, additional treatment and/or surgery may be decided to perform with a certain interval of time, and may be decided to be perform by a certain number of time, based on a result of the detection of the minimal residual disease.

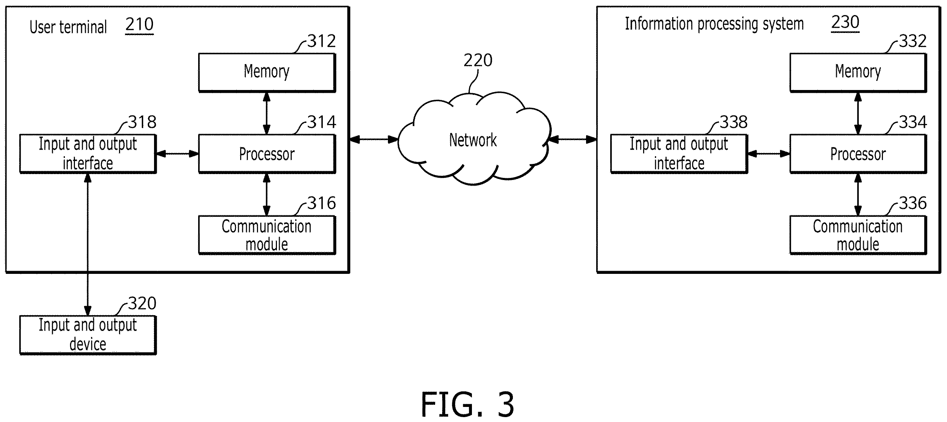

FIG. 3 is a block diagram of an internal configuration of the user terminal 210 and the information processing system 230. The user terminal 210 may refer to any computing device that is capable of executing the application, among other functions, and supporting of wired/wireless communication. Examples include the mobile phone terminal 210_1, the tablet terminal 210_2, and the PC terminal 210_3 as depicted in FIG. 2. As illustrated, the user terminal 210 may include components such as a memory 312, a processor 314, a communication module 316, and an input and output interface 318. Similarly, the information processing system 230 may include components including a memory 332, a processor 334, a communication module 336, and an input and output interface 338. As illustrated in FIG. 3, the user terminal 210 and the information processing system 230 may be configured to communicate information, data, etc. through the network 220 using respective communication modules 316 and 336. In addition, an input and output device 320 may be configured to input information or data to the user terminal 210, or output information or data generated from the user terminal 210 through the input and output interface 318.

The memories 312 and 332 may include any non-transitory computer-readable recording medium. The memories 312 and 332 may include a permanent mass storage device such as read only memory (ROM), disk drive, solid state drive (SSD), flash memory, and others. As another example, a non-destructive mass storage device such as ROM, SSD, flash memory, disk drive, and the like may be included in the user terminal 210 or the information processing system 230 as a separate permanent storage device that is distinct from the memory. In addition, the memories 312 and 332 may store an operating system and at least one program code (e.g., code for the service for detecting minimal residual disease, etc.).

These software components may be loaded from a computer-readable recording medium separate from the memories 312 and 332. Such a separate computer-readable recording medium may include a recording medium directly connectable to the user terminal 210 and the information processing system 230, and may include a computer-readable recording medium such as a floppy drive, a disk, a tape, a DVD/CD-ROM drive, a memory card, and more. As another example, the software components may be loaded into the memories 312 and 332 through the communication modules 316 and 336 rather than from a separate computer-readable recording medium. For example, at least one program may be loaded into the memories 312 and 332 based on a computer program (e.g., applications, etc. associated with the service for detecting minimal residual disease using tumor information) installed by files provided through the network 220 by a developer or by a file distribution system that distributes application installation files.

The processors 314 and 334 may be configured to process the instructions of the computer program by performing basic arithmetic, logic, and input and output operations. The instructions may be provided to the processors 314 and 334 from the memories 312 and 332 or the communication modules 316 and 336. For example, the processors 314 and 334 may be configured to execute the received instructions according to a program code stored in a recording device such as the memories 312 and 332.

The communication modules 316 and 336 may provide a configuration or function for the user terminal 210 and the information processing system 230 to communicate with each other through the network 220, and may provide a configuration or function for the user terminal 210, the information processing system 230, etc. to communicate with another user terminal or another system (e.g., a separate cloud system, etc.). For example, a request or data (e.g., a request to detect a minimal residual disease, a request for genetic data, etc.) generated by the processor 314 of the user terminal 210 according to a program code stored in a recording device such as the memory 312 may be transmitted to the information processing system 230 through the network 220 under the control of the communication module 316. Conversely, a control signal or command provided under the control of the processor 334 of the information processing system 230 may be received by the user terminal 210 through the communication module 316 of the user terminal 210 through the communication module 336 and the network 220.

The input and output interface 318 may serve as a means for interfacing with the input and output device 320. As an example, the input device may include a device such as a camera including an audio sensor and/or an image sensor, a keyboard, a microphone, a mouse, etc., and the output device may include a device such as a display, a speaker, a haptic feedback device, etc. As another example, the input and output interface 318 may be a means for interfacing with a device such as a touch screen, etc. that integrates a configuration or function for performing inputting and outputting. While FIG. 3 illustrates that the input and output device 320 is not included in the user terminal 210, aspects are not limited thereto, and the input and output device 320 may be configured as one device with the user terminal 210. In addition, the input and output interface 338 of the information processing system 230 may be a means for interfacing with a device (not illustrated) for inputting or outputting that may be connected to, or included in the information processing system 230. While FIG. 3 illustrates the input and output interfaces 318 and 338 as the components configured separately from the processors 314 and 334, aspects are not limited thereto, and the input and output interfaces 318 and 338 may be configured to be included in the processors 314 and 334.

The user terminal 210 and the information processing system 230 may include more than those components illustrated in FIG. 3. Meanwhile, it would be unnecessary to exactly illustrate most of the related components. The user terminal 210 may be implemented to include at least a part of the input and output device 320 described above. In addition, the user terminal 210 may further include other components such as a transceiver, a Global Positioning System (GPS) module, a camera, various sensors, a database, etc. For example, if the user terminal 210 is a smartphone, it may generally include components included in the smartphone, and for example, it may be implemented such that various components such as an acceleration sensor, a gyro sensor, a microphone module, a camera module, various physical buttons, buttons using a touch panel, input and output ports, a vibrator for vibration, etc. are further included in the user terminal 210.

The processor 314 of the user terminal 210 may be configured to run an application or a web browser application that provides the service for detecting minimal residual disease using tumor information. In this case, a program code associated with the above application may be loaded into the memory 312 of the user terminal 210. While the application is running, the processor 314 of the user terminal 210 may receive information and/or data provided from the input and output device 320 through the input and output interface 318 or receive information and/or data from the information processing system 230 through the communication module 316, and process the received information and/or data and store it in the memory 312. In addition, such information and/or data may be provided to the information processing system 230 through the communication module 316. For example, the processor 314 may receive genetic data including reference sequence data, sequencing data for normal blood samples of a plurality of sample patients and/or sequencing data for plasma samples from a plurality of sample patients, results of the detection of minimal residual disease, and others from the information processing system 230.

While the application is running, the processor 314 may receive voice data, text, image, video, and the like input or selected through the input device such as a camera, a microphone, and the like that includes a touch screen, a keyboard, an audio sensor and/or an image sensor connected to the input and output interface 318, and store the received voice data, text, image, and/or video or the like in the memory 312, or provide it to the information processing system 230 through the communication module 316 and the network 220.

The processor 314 of the user terminal 210 may transmit and output the information and/or data to the input and output device 320 through the input and output interface 318. For example, the processor 314 of the user terminal 210 may output the processed information and/or data through the output device 320 such as a device capable of outputting a display (e.g., a touch screen, a display, and the like), a device capable of outputting a voice (e.g., speaker), etc.

The processor 334 of the information processing system 230 may be configured to manage, process, and/or store information, data, etc. received from a plurality of user terminals 210, a plurality of external systems, etc. The information and/or data processed by the processor 334 may be provided to the user terminals 210 through the communication module 336 and the network 220.

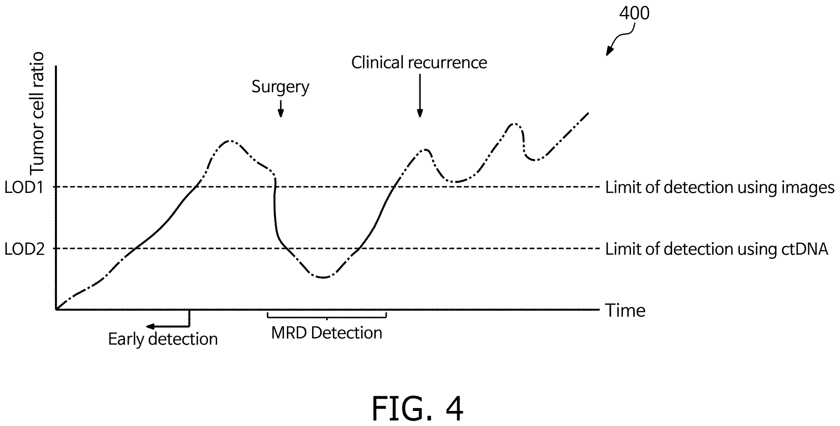

FIG. 4 is a diagram illustrating an example of a graph 400 of the tumor cell fraction in the body of a target patient over time. The graph 400 is an example provided to explain the present disclosure and may represent the tumor cell fraction in the body of a typical tumor patient over time.

In clinical practice, tumor cells in a patient's body may be detected through various methods including medical imaging such as CT scans or through the analysis of ctDNA in the patient's blood. However, there is an important distinction in the sensitivity and timing of detection between these methods. When using medical imaging, tumors typically become visible only when they reach a certain size, which serves as a threshold for detection. This size-based threshold leads to a first limit of detection (LOD1) for tumor cells in the body. In contrast, the analysis of ctDNA allows for the detection of tumor-derived DNA fragments in the bloodstream at earlier stages, even when the tumor size is relatively small. This leads to a lower second limit of detection (LOD2) when using ctDNA. As a result of this difference in sensitivity, ctDNA-based methods can detect tumor cells at lower tumor cell fractions in the body compared to medical imaging. In other words, ctDNA-based methods can potentially detect tumors earlier, before they reach the size threshold visible in medical images. In some embodiments, an image processing using Convolutional Neural Networks (CNNs) can be used to identify the size threshold visible in medical images. A CNN may receive raw medical image data input and then output a set of feature maps representing abstractions of the raw data.

In clinical practice, the first confirmation of a tumor's presence typically occurs through medical imaging, so detecting a tumor before it reaches the LOD1 (i.e., in the interval between LOD1 and LOD2) can be considered an early detection. Early detection of a tumor often leads to interventions such as surgery or treatment to reduce the tumor cell fraction in the body.

However, even after successful surgery or treatment, there may be cases where the tumor cell fraction starts to increase again over time. If this increase surpasses the LOD1, it may be clinically confirmed as tumor recurrence through medical imaging.

The challenge arises when attempting to continuously monitor the presence or absence of tumor cells in the body before clinical recurrence. When the tumor cell fraction is below the LOD2, it becomes challenging to detect tumor cells using ctDNA analysis due to background error rates during the sequencing process. In other words, distinguishing between mutations originating from tumor cells and those resulting from background error rates becomes difficult when the tumor cell fraction is below the LOD2.

To address this challenge, the present disclosure suggests that by reducing the background error rate through data filtering techniques, it's possible to lower the LOD2, allowing for the earlier detection of tumor cells in non-invasive liquid biopsy samples. This approach aims to detect minimal residual disease (MRD) at lower tumor cell fractions, enabling earlier detection while minimizing the burden on patients. In summary, the goal is to enhance the sensitivity of ctDNA-based tumor detection methods, allowing for earlier detection and monitoring of tumor cells, even at lower tumor cell fractions, by reducing background error rates through data filtering.

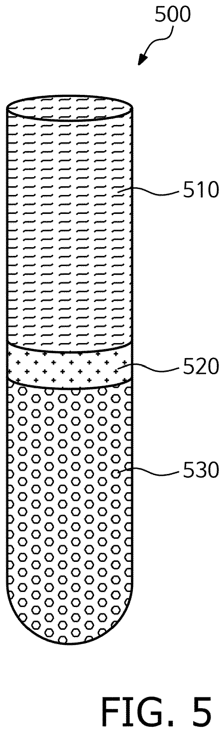

FIG. 5 is a diagram illustrating a blood sample 500 of a target patient. The blood sample 500 may be a blood sample (liquid biopsy sample) obtained by centrifuging the target patient's blood. The centrifuged blood sample 500 may include distinct layers: a top plasma layer 510, a middle normal blood layer 520, and a lower layer 530 containing red blood cells.

The blood sample 500 may be collected from the target patient before or after surgery and/or treatment of the target patient. Specifically, a portion of the normal blood layer 520 may be collected from the blood sample 500 before any surgical procedures or treatments. By conducting whole genome sequencing on this normal blood layer 520, one can acquire normal blood sequencing data. Comparing this normal blood sequencing data to the tumor tissue sequencing data obtained from a biopsy of the patient's tumor allows for the detection of tumor tissue mutations in the target patient.

Following surgery and/or treatment, another portion of the blood sample 500 is collected from the plasma layer 510. The plasma layer 510 may contain cell free Deoxyribo Nucleic Acid (cfDNA) and circulating tumor Deoxyribo Nucleic Acid (ctDNA). Whole genome sequencing is then performed on this plasma layer 510 sample, generating plasma sequencing data. By detecting cfDNA or ctDNA using the plasma sequencing data, it becomes possible to determine various aspects, including the tumor cell fraction within the target patient's body and the presence of tumor recurrence occurred.

FIG. 6 is a diagram illustrating the process of calculating a limit of detection (LOD) 680 for the target patient. The limit of detection 680 for the target patient may represent the minimum tumor cell fraction detectable in the target patient's plasma sample. The limit of detection 680 may vary depending on the number of mutations within the target patient's tumor tissue. The higher the accuracy of the sequencing data acquired through whole genome sequencing, the more accurately the limit of detection 680 may be determined.

As illustrated, the calculation of the limit of detection 680 may rely on tumor tissue sequencing data 610, normal blood sequencing data 620, and plasma sequencing data 660. The tumor tissue sequencing data 610 may be generated through whole genome sequencing (WGS) on a biopsy sample collected from the target patient's tumor. In addition, the normal blood sequencing data 620 may be acquired by conducting WGS on a normal blood sample collected from a normal blood layer (e.g., 520 as seen in FIG. 5) of the blood samples of the target patient. Additionally, the plasma sequencing data 660 may be acquired via WGS from a sample taken from the plasma layer (e.g., 510 as shown in FIG. 5) of the target patient's blood sample.

The limit of detection 680 for the target patient may be calculated based on tumor tissue mutation 630 (specifically, filtered tumor tissue mutation 650) and an average sequencing depth 670 of the plasma sequencing data 660 of the target patient. Specifically, the tumor tissue mutations 630 of the target patient, which are fundamental for calculating the limit of detection 680, may be detected by comparing the tumor tissue sequencing data 610 with the normal blood sequencing data 620. In other words, it may be assumed that normal blood samples do not contain tumor tissue, and thus the normal blood sequencing data 620 may serve as a reference or control sequencing data for identifying genetic mutations in relation to the tumor tissue sequencing data 610. Information regarding the tumor tissue mutation 630 may include details on the location of these mutations and the altered nucleic acid sequences.

Additionally or alternatively, mutations present in ctDNA within plasma can be identified by comparing the normal blood sequencing data 620 and the plasma sequencing data 660. In this scenario, the normal blood sequencing data 620 may serve as a reference or control sequencing data in relation to the plasma sequencing data 660. Mutations that are not detected in the tumor tissue sequencing data 610 but are present in the plasma sequencing data 660 may be subsequently filtered, either replacing or augmenting the tumor tissue mutations 630, and then subject to further filtering 642 and 644. This refined set of mutations may be used to calculate the limit of detection 680 for the target patient.

To generate the filtered tumor tissue mutation 650 from the detected tumor tissue mutation 630, a background error filtering process, including filtering 642 and 644, may be performed. This process may utilize genetic data associated with a plurality of sample patients who are distinct from the target patient for detecting minimal residual disease.

For example, if the target patient is afflicted with lung cancer, a set of 33 lung cancer patients, distinct from the target patient are chosen as a plurality of sample patients for this purpose. The background error filtering may leverage the genetic data of these 33 lung cancer patients, including their normal blood sequencing data and plasma sequencing data. Additionally, their tumor tissue sequencing data may be further leveraged. The genetic data associated with the plurality of sample patients used for the filtering 642 and 644 may be acquired from a genetic database 640.

The first filtering 642 of the tumor tissue mutations 630 using the normal blood sequencing data derived from the plurality of sample patients' samples. Specifically, during the first filtering 642, mutations detected in the sequencing data of the normal blood samples taken from the plurality of sample patients may be removed from the detected tumor tissue mutations 630. That is, these mutations previously identified from normal blood samples of the plurality of sample patients may be considered errors rather than genuine tumor tissue mutations and may thus be removed during the first filtering 642.

Subsequently, the second filtering 644 may be applied to the tumor tissue mutations that have undergone the first filtering 642. The second filtering 644 may rely on the sequencing data from the plasma samples obtained from the plurality of sample patients. In particular, among the detected tumor tissue mutations, those matching mutations found in the plasma sample sequencing data of the plurality of sample patients, may be removed from the tumor tissue mutation information, which had previously undergone the first filtering 642. That is, these mutations, which align with mutations identified in the sequencing data of the plasma samples from the plurality of sample patients, may be regarded as system errors and removed from the tumor tissue mutations that have undergone the first filtering 642.

In FIG. 6, it is illustrated and explained that the second filtering 644 is performed after the first filtering 642 is performed, but this sequence is not the only possible order. For example, the second filtering 644 described above may be performed first, and then the first filtering 642 described above may be performed. In other examples, either the first filtering 642 or the second filtering 644 may be omitted.

Meanwhile, the average sequencing depth 670 of the ctDNA or the plasma sequencing data 660 within the target patient's blood may be calculated based on the plasma sequencing data 660 obtained from the target patient. Using the filtered tumor tissue mutation 650 information and the calculated average sequencing depth 670 of the plasma sequencing data 660, the limit of detection 680 may be determined. For example, the limit of detection 680 may be calculated based on Equation 1 below.

<Equation 1>

$$LOD = 1 - (1 - \frac{1}{N})^{1/cov}$$

where, N may represent the number of mutations detected in tumor tissue (e.g., the number of filtered tumor tissue mutations), and *cov* may represent the average sequencing depth of the plasma sequencing data.

FIG. 7 is a diagram illustrating a process of tumor detection 780. As illustrated, a tumor cell fraction (TCF) 730 for the target patient may be calculated based on plasma sequencing data 710 and a reference sequence 720. In addition, a corrected tumor cell fraction 760 may be calculated using a random error rate 750. Tumor detection 780 may then be performed based on the corrected tumor cell fraction 760 and a patient's limit of detection (LOD) 770 of the patient. The limit of detection 770 may be calculated in similar way as the limit of detection 680 calculated for the target patient in FIG. 6.

The tumor cell fraction 730 may be determined based on the plasma sequencing data 710 and the reference sequence 720. Specifically, in the plasma sequencing data 710, the number of reads that differ from the reference sequence 720 and the number of reads that match the reference sequence 720 may be determined, respectively. The tumor cell fraction 730 may be calculated based on the number of reads that differ from the reference sequence 720 and the number of reads that match the reference sequence 720. For example, the tumor cell fraction 730 may be calculated by Equation 2 below

<Equation 2>

$$Tumor\ Cell\ Fraction\ (raw) = \frac{\sum alternative\ allele\ count}{\sum(alternative\ allele\ count + reference\ allele\ count)}$$

where, *Tumor Cell Fraction* (*raw*) may represent the tumor cell fraction 730, $\Sigma$ *alternative allele count* may represent the number of reads different from the reference sequence 720, and $\Sigma$ *reference allele count* may represent the number of reads that match the reference sequence 720.

Alternatively, the tumor cell fraction 730 may be calculated based on the filtered tumor tissue mutation 650 of FIG. 6. Since the filtered tumor tissue mutation 650 information in FIG. 6 includes information associated with the region where the mutation occurs in the genome of the tumor cell, the number of reads including the mutation detected in the tumor tissue may be calculated using the filtered tumor tissue mutation 650.

Specifically, the number of reads in the patient's plasma sequencing data including the mutations detected in the tumor tissue may be determined based on the filtered tumor tissue mutation information, and the determined number of reads may be used as $\Sigma$ *alternative allele count* in Equation 2. For example, if there are 2000 filtered tumor tissue mutations, the regions corresponding to these 2,000 tumor tissue mutations may be examined in the plasma sequencing data to identify the presence of mutations. For example, if ctDNA in the plasma is sequenced at 20x coverage, the number of reads including mutations detected in tumor tissue may be determined as 20 * 2,000 = 40,000 reads. The number of reads containing mutations from the tumor tissue in the plasma sequencing data, as determined using the filtered tumor tissue mutation 650 information of FIG. 6, is expected to be substantially the same as or very close to the number of reads different from the reference sequence 720. Through this, the time and computing resources required to compare the entire plasma sequencing data 710 with the reference sequence 720 may be reduced.

Meanwhile, the random error rate 750 may be calculated based on the genetic data and the reference sequences 720 associated with the plurality of sample patients distinct from the target patient. The genetic data associated with the plurality of sample patients may be acquired from a genetic database 740. In some embodiments, an automated machine learning scheme is used for generating and updating the genetic data of the genetic database 740. In some embodiments, a hardware device performs, through the automated machine learning scheme, which use automating machine learning workflows for genomics, generating and updating the genetic data of the genetic database 740, with a certain interval of time, automatically. The genetic database 740 may be the same database as the genetic database 640 of FIG. 6.

The genetic data in the genetic database 740 associated with the plurality of sample patients may include their plasma sequencing data of the plurality of sample patients. The number of reads differing from the reference sequence 720 and the number of reads matching the reference sequence 720 may be determined within the plasma sequencing data of these sample patients. Subsequently, the random error rate 750 may be calculated based on each number of reads. For example, the random error rate 750 may be calculated based on Equation 3 below

<Equation 3>

$$Random\ Error\ Rate = \frac{\sum alternative\ allele\ count}{\sum(alternative\ allele\ count + reference\ allele\ count)}$$

where, *Random Error Rate* represents the random error rate 750, $\Sigma$ *alternative allele count* may represent the number of reads different from the reference sequence 720 in the plasma sequencing data of the plurality of sample patients, and $\Sigma$ *reference allele count* may represent the number of reads that match the reference sequence 720 in the plasma sequencing data of the plurality of sample patients.

The tumor cell fraction 730 may be corrected using the random error rate 750. For example, the corrected tumor cell fraction 760 may be calculated by subtracting the random error rate 750 from the tumor cell fraction 730.

The tumor detection 780 for the target patient (e.g., determining tumor recurrence, etc.) may be performed based on the corrected tumor cell fraction 760 and the limit of detection 770. For example, a confidence interval with a predetermined confidence level may be established for the corrected tumor cell fraction 760. Using a 95% confidence level as an example, the confidence interval at the 95% confidence level may be calculated using Equation 4 below

<Equation 4>

$$95\% \text{ confidence interval } = TCF \pm 1.96 \sqrt{\frac{TCF(1 - TCF)}{D}}$$

where, TCF represents the corrected tumor cell fraction 760.

In response to determining that the lower limit of the confidence interval of the calculated corrected tumor cell fraction 760 surpasses the limit of detection 770, it may be concluded that the patient's tumor has recurred (the tumor detection 780). If tumor recurrence is established, tumor treatment and/or surgery may be performed on the target patient.

Unlike what is illustrated and described in FIG. 7, the tumor detection 780 may be performed utilizing structural variants. The determination of whether the target patient has experienced tumor recurrence may be based on a comparison between a first set of structural variants detected from the tumor tissue sequencing data and a second set of structural variants detected from the plasma sequencing data. For example, if the number or ratio of structural variants within the second set of structural variants, relative to the first set of structural variants equals or exceeds a predetermined threshold (e.g., 0.9), it may be determined that the patient's tumor has recurred. The first set of structural variants may include types such as inversion, translocation, duplication, deletion, or insertion occurring in specific regions of the genome.

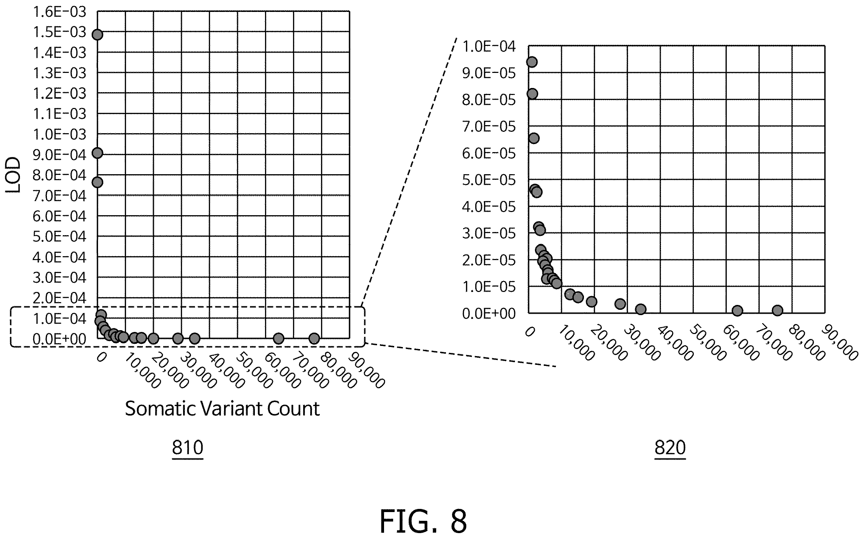

FIG. 8 is a diagram illustrating example graphs 810 and 820 showing the relationship between the number of mutations detected in the tumor cells of the target patient and the limit of detection for the target patient. The graphs 810 and 820 represent actual measurement results of the limit of detection for each sample patient, based on the ctDNA samples from the 33 sample patients. Specifically, the first graph 810 represents the limits of detection for all 33 sample patients, while the second graph 820 represents the limits of detection within a specific range observed in the first graph 810.

In the first graph 810, the limits of detection for all sample patients fall within a range of approximately 1.48E-06 to 1.49E-03, with a median value roughly 1.99E-05. Notably, 29 out of the 33 sample patients (approximately 87% of the sample patients) have limits of detection less than 1E-4. For sample patients where ctDNA matches tumor cells containing more than 20,000 mutations, the limit of detection drops to 1E-6. This demonstrates a correlation between a sample patient's limit of detection and the somatic variant count of within their tumor cells. As the somatic variant count increases, the limit of detection, representing the lowest detectable tumor cell fraction in the patent's tumor plasma sample, tends to decrease, making tumor detection easier.

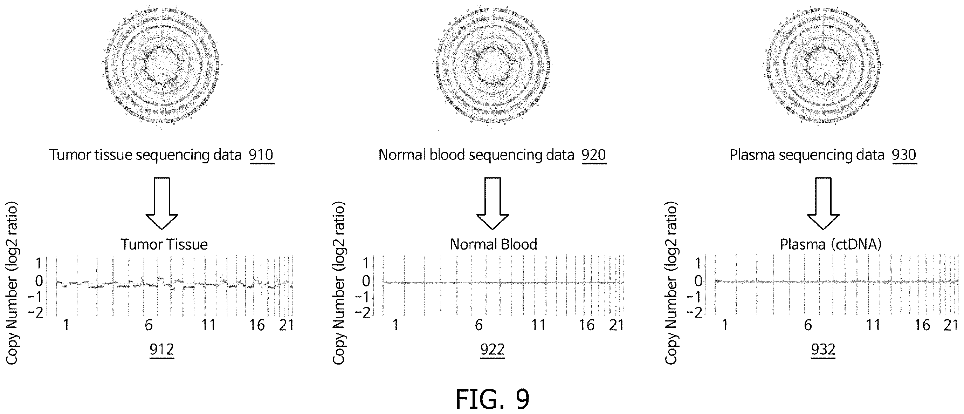

FIG. 9 is a diagram illustrating an example of generating arm-level copy number profiles 912, 922, and 932. Additionally or alternatively to the methods described above in FIGS. 6 and 7, the minimal residual disease may be detected using arm-level copy number profile.

The first chromosomal arm-level copy number profile 912 may be generated from tumor tissue sequencing data 910 of the target patient, the second chromosomal arm-level copy number profile 922 may be generated from normal blood sequencing data 920, and the third chromosomal arm-level copy number profile 932 may be generated from plasma sequencing data 930. The tumor tissue sequencing data 910, the normal blood sequencing data 920, and the plasma sequencing data 930 may be acquired from each of the tumor tissue biopsy sample, the normal blood sample, and the plasma sample of the target patient.

To generate the arm-level copy number profiles 912, 922, and 932 each set of the sequencing data 910, 920, and 930 may be mapped to a reference genome. For example, for each of the sequencing data 910, 920, and 930, the information on the genomic position of the chromosomal arm may be acquired, the sequencing coverage (or sequencing depth or number of reads) of the reads mapped to each chromosomal arm may be counted, and the whole genome sequencing coverage of WGS data may be divided, thereby acquiring the normalized copy number profiles 912, 922, and 932 at the chromosomal arm-level.

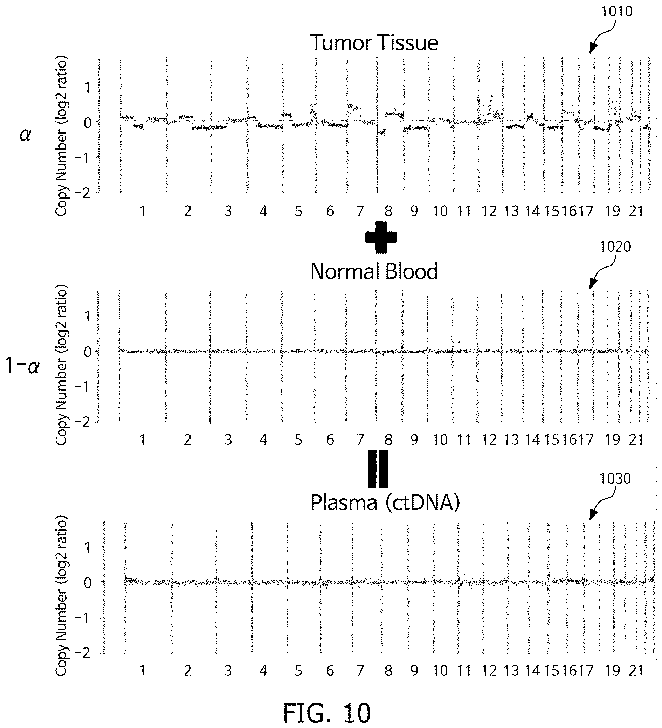

FIG. 10 is a diagram illustrating an example of calculating the tumor cell fraction in the target patient's body using the arm-level copy number profiles 1010, 1020, and 1030. The third chromosomal arm-level copy number profile 1030, derived from the plasma sequencing data of the target patient may be considered as a mixture in a specific ratio of the first chromosomal arm-level copy number profile 1010, generated from the tumor tissue sequencing data of the target patient, and the second chromosomal arm-level copy number profile 1020, produced from the normal blood sequencing data of the target patient. For example, as illustrated, the first chromosomal arm-level copy number profile 1010 and the second chromosomal arm-level copy number profile 1020 may be considered to be mixed at a ratio of $\alpha$: (1-$\alpha$), where $\alpha$ is a real number between 0 and 1, representing the tumor cell fraction (TCF) (e.g., 730 in FIG. 7) for the patient. The $\alpha$ value (i.e., tumor cell fraction) may be calculated using the Non-Negative Least Squares (NNLS) method expressed in Equation 5

below.

<Equation 5>

$$argmin_x \left\| Ax - y \right\|_2^2 \; subject \; to \; x \geq 0$$

where, $\|Ax\text{-}y\|_2$ may represent the Euclidean norm, and A may be a matrix associated with the first chromosomal arm-level copy number profile 1010 and the second chromosomal arm-level copy number profile 1020, and y may be a vector associated with the third chromosomal arm-level copy number profile 1030.

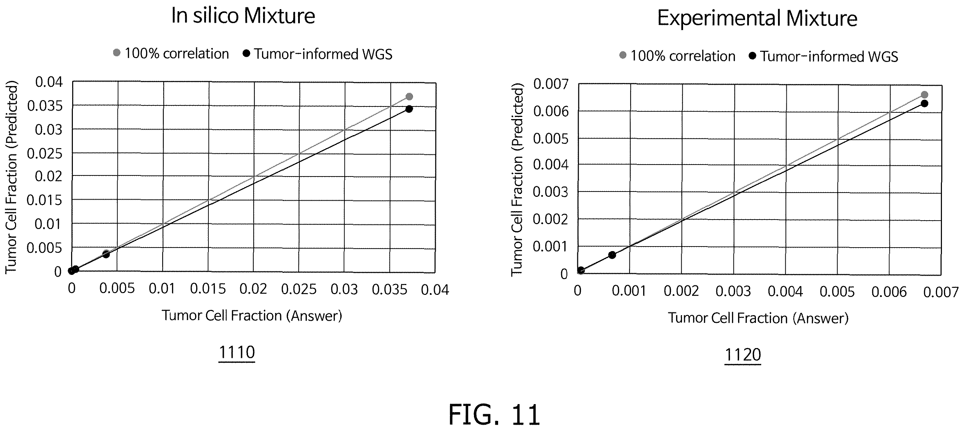

FIG. 11 is a diagram illustrating performance verification results of a method for detecting minimal residual disease in a target patient using the genetic data of the sample patient. In the first graph 1110, the results of mixing tumor tissue sequencing data and normal blood sequencing data with known tumor cell fractions at various ratios (in silico mixture) are presented, verifying the accuracy of the method for detecting residual disease using the genetic data of the sample patient(e.g., the method illustrated and described in FIGS. 6 and 7) in measuring the Tumor Cell Fraction (TCF). The second graph 1120 shows the results of mixing actual tumor tissue DNA with normal DNA (experimental mixture) and verifying how accurately the method for detecting residual disease using sample patient data can measure the tumor cell fraction.

As illustrated in both the first graph 1110 and the second graph 1120, in cases of mixing sequencing data and mixing DNA, it is confirmed that there is a correlation of 99.9% or more between the correct tumor cell fraction (Answer) and the predicted tumor cell fraction (Predicted) according to the present disclosure. In other words, as a result of filtering the tumor tissue mutation of the target patient using the genetic data of a sample patient, it can be confirmed that the tumor cell fraction in the target patient's body can be predicted very accurately.

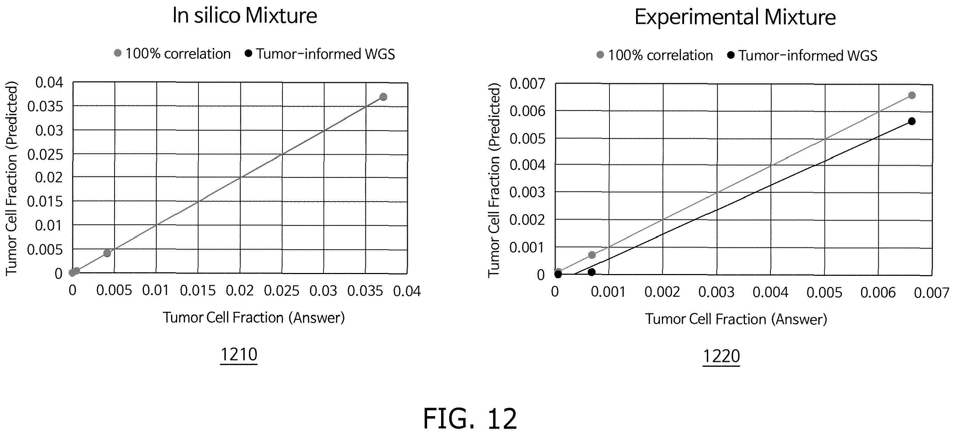

FIG. 12 is a diagram illustrating performance verification results of a method for detecting minimal residual disease in a target patient using a chromosomal arm-level copy number profile of the target patient. Similar to FIG. 11, the performance verification of the method (e.g., the method illustrated and described in FIGS. 9 and 10) for detecting minimal residual disease in the target patient using the chromosomal arm-level copy number profile of the target patient is performed on in silico mixture (the first graph 1210) and experimental mixture (the second graph 1220).

As illustrated in both the first graph 1210 and the second graph 1220, in cases of mixing sequencing data and mixing DNA, it is confirmed that there is a correlation of 99.9% or more between the correct tumor cell fraction (Answer) and the predicted tumor cell fraction (Predicted) according to the present disclosure. In other words, it can be confirmed that the tumor cell fraction in the target patient's body can be predicted very accurately by using the chromosomal arm-level copy number profile of the target patient.

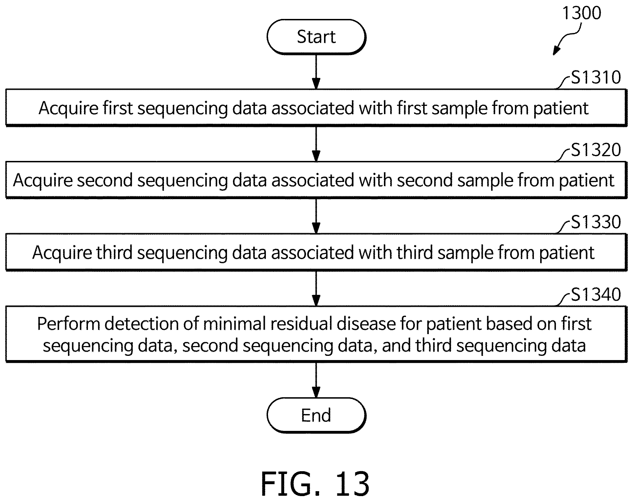

FIG. 13 is a flowchart illustrating a method 1300 for detecting minimal residual disease using tumor information. The method 1300 may be performed by one or more processors of a system for detecting minimal residual disease using tumor information. The method 1300 may begin by the processor acquiring first sequencing data associated with a first sample from a patient, at S1310. The first sample may be a patient's tumor tissue biopsy sample, and the first sequencing data may be obtained through whole genome sequencing (WGS).

Subsequently, at S1320, the processor may acquire second sequencing data associated with a second sample from the patient. This second sample is a normal blood sample from the patient, and the second sequencing data may be acquired through whole genome sequencing.

Continuing with the method, at S1330, the processor may acquire third sequencing data associated with a third sample from the patient. The third sample is a plasma sample derived from the patient, and this plasma sample may include cell free Deoxyribo Nucleic Acid (cfDNA) and circulating tumor Deoxyribo Nucleic Acid (ctDNA). The third sequencing data may be acquired through whole genome sequencing.

It's noteworthy that the first and second samples may be obtained at a first time point, while the third sample may be obtained at a second time point after the first time point. Furthermore, the patient may have undergone at least one of surgery or therapy after the first time point and before the second time point.

The processor may detect tumor tissue mutation information of the patient by comparing the first sequencing data and the second sequencing data, perform background error filtering on the detected tumor tissue mutation information using genetic data associated with a plurality of sample patients that are distinct from the patient. This genetic data may include sequencing data for normal blood samples from a plurality of sample patients and/or sequencing data for plasma samples from a plurality of sample patients.

The processor may perform background error filtering by removing, from the tumor tissue mutation information, the mutations among the detected tumor tissue mutations that were also found in the sequencing data for the normal blood samples of the plurality of sample patients. In another example, the processor may perform background error filtering by removing, from the tumor tissue mutation information, the mutations detected in the sequencing data for the plasma samples of the plurality of sample patients.

The processor may perform detection of minimal residual disease for the patient based on the first sequencing data, the second sequencing data, and the third sequencing data, at S 1340. The processor may calculate a limit of detection for the patient based on the first sequencing data, the second sequencing data, and the third sequencing data, calculate a tumor cell fraction (TCF) for the patient based on the third sequencing data, correct the tumor cell fraction using genetic data associated with the plurality of sample patients distinct from the patient, and determine whether the patient has tumor recurrence based on the corrected tumor cell fraction and the limit of detection, thereby performing detection of minimal residual disease for the patient. The limit of detection may represent the lowest tumor cell fraction detectable in the patient's plasma sample. In addition, the limit of detection may be calculated based on the number of tumor tissue mutations detected in the patient by comparing the first sequencing data and the second sequencing data, along with an average sequencing depth of the third sequencing data.

Additionally, the processor may determine the number of reads that differ from a reference sequence in the third sequencing data and the number of reads that match the reference sequence in the third sequencing data. The tumor cell fraction may be calculated based on these read counts.

Moreover, the processor may detect tumor tissue mutation information of the patient by comparing the first sequencing data and the second sequencing data. It may then determine the number of reads in the third sequencing data, including mutations detected in the tumor tissue, based on the tumor tissue mutation information, and employ this count as the number of reads that differ from the reference sequence.

Furthermore, the processor may determine the number of reads that differ from a reference sequence in plasma sequencing data included in genetic data associated with a plurality of sample patients distinct from the patient, determine the number of reads that match the reference sequence in the plasma sequencing data included in the genetic data associated with the plurality of sample patients distinct from the patient, calculate a random error rate based on the number of reads that differ from the reference sequence and the number of reads that match the reference sequence. This random error rate is used to correct the tumor cell fraction.

The processor may calculate a confidence interval with a predetermined confidence level for the corrected tumor cell fraction. If it determines that the lower limit of the calculated confidence interval is higher than the calculated limit of detection, the processor may conclude that the patient's tumor has recurred.

The processor, in an alternative approach, may identify a first set of structural variants detected from the first sequencing data, and a second set of structural variants detected from the third sequencing data. It may then determine whether the patient has tumor recurrence based on a comparison result between these two sets of structural variants. For example, the processor may determine that the patient's tumor has recurred if the number or ratio of structural variants found in the second set among the first set of structural variants equals or exceeds a threshold. The first set of structural variants may include at least one of inversion, translocation, duplication, deletion, or insertion in some regions of the genome.

Alternatively, the processor may perform detection of minimal residual disease by generating a first chromosomal arm-level copy number profile based on the first sequencing data, generating a second chromosomal arm-level copy number profile based on the second sequencing data, generating a third chromosomal arm-level copy number profile based on the third sequencing data, and calculating the tumor cell fraction based on these first to third chromosomal arm-level copy number profiles.

The flowchart illustrated in FIG. 13 and the above description are merely examples and may be implemented differently in some examples. For example, one or more operations may be omitted, the order of operations may be changed, one or more operations may be performed in parallel, or one or more operations may be repeatedly performed multiple times.

Further, in some embodiments, the flowchart illustrated in FIG. 13 is performed by one hardware medical system. In those embodiments, the hardware medical system has one or more medical proves configured to acquire the first sample, the second sample, and the third sample, and also has the processor, that is a hardware and acquires the first sequencing data, the second sequencing data, and the third sequencing data, associated with the first sample, the second sample, and the third sample, respectively. The hardware medical system according to those embodiments also has a hardware memory storing data to be used for detecting of minimal residual disease for the patient based on the first sequencing data, the second sequencing data, and the third sequencing data. The hardware memory also store data regarding a type of additional treatment and/or surgery, which should be conducted for the target patient and matched with the results of the detection. In those embodiments, the hardware medical system includes one or more medical devices configured to perform the additional treatment and/or surgery, for example, a robot arm.

The method described above may be provided as a computer program stored in a computer-readable recording medium for execution on a computer. The medium may be a type of medium that continuously stores a program executable by a computer, or temporarily stores the program for execution or download. In addition, the medium may be a variety of writing means or storage means having a single piece of hardware or a combination of several pieces of hardware, and is not limited to a medium that is directly connected to any computer system, and accordingly, may be present on a network in a distributed manner. An example of the medium includes a medium configured to store program instructions, including a magnetic medium such as a hard disk, a floppy disk, and a magnetic tape, an optical medium such as a CD-ROM and a DVD, a magnetic-optical medium such as a floptical disk, and a ROM, a RAM, a flash memory, etc. In addition, other examples of the medium may include an app store that distributes applications, a site that supplies or distributes various software, and a recording medium or a storage medium managed by a server.

The methods, operations, or techniques of the present disclosure may be implemented by various means. For example, these techniques may be implemented in hardware, firmware, software, or a combination thereof. Those skilled in the art will further appreciate that various illustrative logical blocks, modules, circuits, and algorithm steps described in connection with the disclosure herein may be implemented in electronic hardware, computer software, or combinations of both. To clearly illustrate this interchangeability of hardware and software, various illustrative components, blocks, modules, circuits, and steps have been described above generally in terms of their functionality. Whether such a function is implemented as hardware or software varies according to design requirements imposed on the particular application and the overall system. Those skilled in the art may implement the described functions in varying ways for each particular application, but such implementation should not be interpreted as causing a departure from the scope of the present disclosure.

In a hardware implementation, processing units used to perform the techniques may be implemented in one or more ASICs, DSPs, digital signal processing devices (DSPDs), programmable logic devices (PLDs), field programmable gate arrays (FPGAs), processors, controllers, microcontrollers, microprocessors, electronic devices, other electronic units designed to perform the functions described in the present disclosure, computer, or a combination thereof.

Accordingly, various example logic blocks, modules, and circuits described in connection with the present disclosure may be implemented or performed with general purpose processors, DSPs, ASICs, FPGAs or other programmable logic devices, discrete gate or transistor logic, discrete hardware components, or any combination of those designed to perform the functions described herein. The general purpose processor may be a microprocessor, but in the alternative, the processor may be any related processor, controller, microcontroller, or state machine. The processor may also be implemented as a combination of computing devices, for example, a DSP and microprocessor, a plurality of microprocessors, one or more microprocessors associated with a DSP core, or any other combination of the configurations.

In the implementation using firmware and/or software, the techniques may be implemented with instructions stored on a computer-readable medium, such as random access memory (RAM), read-only memory (ROM), non-volatile random access memory (NVRAM), programmable read-only memory (PROM), erasable programmable read-only memory (EPROM), electrically erasable PROM (EEPROM), flash memory, compact disc (CD), magnetic or optical data storage devices, etc. The instructions may be executable by one or more processors, and may cause the processor(s) to perform certain aspects of the functions described in the present disclosure.

Although the examples described above have been described as utilizing aspects of the currently disclosed subject matter in one or more standalone computer systems, aspects are not limited thereto, and may be implemented in conjunction with any computing environment, such as a network or distributed computing environment. Furthermore, the aspects of the subject matter in the present disclosure may be implemented in multiple processing chips or apparatus, and storage may be similarly influenced across a plurality of apparatus. Such apparatus may include PCs, network servers, and portable apparatus.

Although the present disclosure has been described in connection with some examples herein, various modifications and changes can be made without departing from the scope of the present disclosure, which can be understood by those skilled in the art to which the present disclosure pertains. In addition, such modifications and changes should be considered within the scope of the claims appended herein.

**Claims**

1. A method for detecting minimal residual disease, the method being performed by one or more processors and comprising:

   acquiring first sequencing data associated with a first sample from a patient;
   acquiring second sequencing data associated with a second sample from the patient;
   acquiring third sequencing data associated with a third sample from the patient; and
   performing detection of minimal residual disease for the patient based on the first sequencing data, the second sequencing data, and the third sequencing data.

2. The method according to claim 1, wherein the first sample is a tumor tissue biopsy sample of the patient,

   the second sample is a normal blood sample of the patient,

the third sample is a plasma sample of the patient, and
the plasma sample includes cell free Deoxyribo Nucleic Acid (cfDNA) and circulating tumor Deoxyribo Nucleic Acid (ctDNA).

3. The method according to claim 1, wherein the first sample is acquired at a first time point,

the third sample is acquired at a second time point after the first time point, and
at least one of surgery or therapy is performed on the patient after the first time point and before the second time point.

4. The method according to claim 1, further comprising:

detecting tumor tissue mutation information of the patient by comparing the first sequencing data and the second sequencing data; and
performing background error filtering on the detected tumor tissue mutation information using genetic data associated with a plurality of sample patients who are distinct from the patient.

5. The method according to claim 4, wherein the genetic data includes sequencing data for normal blood samples of the plurality of sample patients, and
the performing the filtering includes removing, mutations among the detected tumor tissue mutations, which are detected in sequencing data for the normal blood samples of the plurality of sample patients from the tumor tissue mutation information.

6. The method according to claim 4, wherein the genetic data includes sequencing data for plasma samples from the plurality of sample patients, and
the performing the filtering includes removing, of the detected tumor tissue mutations, mutations detected in sequencing data for the plasma samples from the plurality of sample patients from the tumor tissue mutation information.

7. The method according to claim 1, wherein the performing the detection of minimal residual disease includes:

calculating a limit of detection for the patient based on the first sequencing data, the second sequencing data, and the third sequencing data;
calculating a tumor cell fraction (TCF) for the patient based on the third sequencing data;
correcting the tumor cell fraction using genetic data associated with a plurality of sample patients distinct from the patient; and
determining whether the patient has tumor recurrence based on the corrected tumor cell fraction and the limit of detection.

8. The method according to claim 7, wherein the limit of detection represents the lowest tumor cell fraction that can be detected in a plasma sample of the patient.

9. The method according to claim 7, wherein the limit of detection is calculated based on the number of tumor tissue mutations of the patient detected by comparing the first sequencing data and the second sequencing data and an average sequencing depth of the third sequencing data.

10. The method according to claim 7, wherein the calculating the tumor cell fraction includes:

determining the number of reads that are different from a reference sequence in the third sequencing data;
determining the number of reads that match the reference sequence in the third sequencing data; and
calculating the tumor cell fraction based on the number of reads that are different from the reference sequence and the number of reads that match the reference sequence.

11. The method according to claim 10, wherein the determining the number of reads that are different from the reference sequence includes:

detecting tumor tissue mutation information of the patient by comparing the first sequencing data and the second sequencing data;

determining the number of reads in the third sequencing data including mutations detected in tumor tissue based on the tumor tissue mutation information; and

using the number of reads in the third sequencing data including the mutations detected in the tumor tissue as the number of reads that are different from the reference sequence.

12. The method according to claim 7, wherein the correcting the tumor cell fraction includes:

determining the number of reads that are different from a reference sequence in plasma sequencing data included in genetic data associated with a plurality of sample patients distinct from the patient;

determining the number of reads that match the reference sequence in the plasma sequencing data included in the genetic data associated with the plurality of sample patients distinct from the patient;

calculating a random error rate based on the number of reads that are different from the reference sequence and the number of reads that match the reference sequence; and

correcting the tumor cell fraction using the random error rate.

13. The method according to claim 7, wherein the determining whether the patient has tumor recurrence includes:

calculating a confidence interval of a predetermined confidence level of the corrected tumor cell fraction; and

in response to determining that a lower limit of the calculated confidence interval is higher than the calculated limit of detection, determining that the patient's tumor has recurred.

14. The method according to claim 3, wherein the performing the detection of minimal residual disease further includes:

identifying a first set of structural variants detected from the first sequencing data;

identifying a second set of structural variants detected from the third sequencing data; and

determining whether the patient has tumor recurrence based on a comparison result of the first set of structural variants and the second set of structural variants,

wherein the determining whether the patient has tumor recurrence includes determining that the tumor has recurred in the patient if the number or ratio of structural variants included in the second set of structural variants among the first set of structural variants is equal to or greater than a threshold.

15. A system comprising:

a communication module;

a memory; and

one or more processors connected to the memory and configured to execute one or more computer-readable programs included in the memory, wherein the one or more programs include instructions for:

acquiring first sequencing data associated with a first sample from a patient;

acquiring second sequencing data associated with a second sample from the patient;

acquiring third sequencing data associated with a third sample from the patient; and

performing detection of minimal residual disease for the patient based on the first sequencing data, the second sequencing data, and the third sequencing data.

**Amended claims in accordance with Rule 137(2) EPC.**

1. A method for detecting minimal residual disease, the method being performed by one or more processors and comprising:

acquiring first sequencing data associated with a first sample from a patient, wherein the first sample is a tumor tissue biopsy sample of the patient, and wherein the first sample is acquired at a first time point;

acquiring second sequencing data associated with a second sample from the patient, wherein the second sample is a normal blood sample of the patient;

acquiring third sequencing data associated with a third sample from the patient, wherein the third sample is a plasma sample of the patient, and wherein the third sample is acquired at a second time point after the first time point, and at least one of surgery or therapy is performed in the patient after the first time point and before the second time point; and

performing detection of minimal residual disease for the patient based on the first sequencing data, the second sequencing data, and the third sequencing data, wherein the performing the detection of minimal residual disease includes:

calculating a limit of detection based on the number of tumor tissue mutations of the patient detected by comparing the first sequencing data and the second sequencing data and an average sequencing depth of the third sequencing data;
calculating a tumor cell fraction (TCF) for the patient based on the third sequencing data;
correcting the tumor cell fraction using genetic data associated with a plurality of sample patients distinct from the patient; and
determining whether the patient has tumor recurrence based on the corrected tumor cell fraction and the limit of detection.

2. The method according to claim 1, wherein the plasma sample includes cell free Deoxyribo Nucleic Acid (cfDNA) and circulating tumor Deoxyribo Nucleic Acid (ctDNA).

3. The method according to claim 1, further comprising:
detecting tumor tissue mutation information of the patient by comparing the first sequencing data and the second sequencing data; and
performing background error filtering on the detected tumor tissue mutation information using genetic data associated with a plurality of sample patients who are distinct from the patient.

4. The method according to claim 3, wherein the genetic data includes sequencing data for normal blood samples of the plurality of sample patients, and
the performing the filtering includes removing, mutations among the detected tumor tissue mutations, which are detected in sequencing data for the normal blood samples of the plurality of sample patients from the tumor tissue mutation information.

5. The method according to claim 3, wherein the genetic data includes sequencing data for plasma samples from the plurality of sample patients, and
the performing the filtering includes removing, of the detected tumor tissue mutations, mutations detected in sequencing data for the plasma samples from the plurality of sample patients from the tumor tissue mutation information.

6. The method according to claim 1, wherein the limit of detection represents the lowest tumor cell fraction that can be detected in a plasma sample of the patient.

7. The method according to claim 1, wherein the calculating the tumor cell fraction includes:

determining the number of reads that are different from a reference sequence in the third sequencing data;
determining the number of reads that match the reference sequence in the third sequencing data; and
calculating the tumor cell fraction based on the number of reads that are different from the reference sequence and the number of reads that match the reference sequence.

8. The method according to claim 7, wherein the determining the number of reads that are different from the reference sequence includes:
detecting tumor tissue mutation information of the patient by comparing the first sequencing data and the second sequencing data;

determining the number of reads in the third sequencing data including mutations detected in tumor tissue based on the tumor tissue mutation information; and
using the number of reads in the third sequencing data including the mutations detected in the tumor tissue as the number of reads that are different from the reference sequence.

9. The method according to claim 1, wherein the correcting the tumor cell fraction includes:

determining the number of reads that are different from a reference sequence in plasma sequencing data included in the genetic data associated with the plurality of sample patients distinct from the patient;
determining the number of reads that match the reference sequence in the plasma sequencing data included in the genetic data associated with the plurality of sample patients distinct from the patient;

calculating a random error rate based on the number of reads that are different from the reference sequence and the number of reads that match the reference sequence; and

correcting the tumor cell fraction using the random error rate.

10. The method according to claim 1, wherein the determining whether the patient has tumor recurrence includes:

calculating a confidence interval of a predetermined confidence level of the corrected tumor cell fraction; and

in response to determining that a lower limit of the calculated confidence interval is higher than the calculated limit of detection, determining that the patient's tumor has recurred.

11. A system comprising:

a communication module;

a memory; and

one or more processors connected to the memory and configured to execute one or more computer-readable programs included in the memory, wherein the one or more programs include instructions for:

acquiring first sequencing data associated with a first sample from a patient, wherein the first sample is a tumor tissue biopsy sample of the patient, and wherein the first sample is acquired at a first time point;

acquiring second sequencing data associated with a second sample from the patient, wherein the second sample is a normal blood sample of the patient;

acquiring third sequencing data associated with a third sample from the patient, wherein the third sample is a plasma sample of the patient, and wherein the third sample is acquired at a second time point after the first time point, and at least one of surgery or therapy is performed in the patient after the first time point and before the second time point; and

performing detection of minimal residual disease for the patient based on the first sequencing data, the second sequencing data, and the third sequencing data,

wherein the performing the detection of minimal residual disease includes:

calculating a limit of detection based on the number of tumor tissue mutations of the patient detected by comparing the first sequencing data and the second sequencing data and an average sequencing depth of the third sequencing data;

calculating a tumor cell fraction (TCF) for the patient based on the third sequencing data;

correcting the tumor cell fraction using genetic data associated with a plurality of sample patients distinct from the patient; and

determining whether the patient has tumor recurrence based on the corrected tumor cell fraction and the limit of detection.

FIG. 1

210_1

210_2

220

230

Network

Information
processing system

210_3

FIG. 2

FIG. 3

FIG. 4

FIG. 5

Tumor tissue sequencing data 610

Normal blood sequencing data 620

Plasma sequencing data 660

Tumor tissue mutation 630

Average sequencing depth 670
640

First filtering 642

DB

Second filtering 644

Filtered tumor tissue mutation 650

LOD of patient 680

FIG. 6

FIG. 7

FIG. 8

FIG. 9

FIG. 10

FIG. 11

**In silico Mixture**

100% correlation · Tumor-informed WGS

1210

**Experimental Mixture**

100% correlation · Tumor-informed WGS

1220

FIG. 12

EP 4 513 496 A1

1300

Start

S1310

Acquire first sequencing data associated with first sample from patient

S1320

Acquire second sequencing data associated with second sample from patient

S1330

Acquire third sequencing data associated with third sample from patient

S1340

Perform detection of minimal residual disease for patient based on first sequencing data, second sequencing data, and third sequencing data

End

FIG. 13

| | Europäisches Patentamt European Patent Office Office européen des brevets | **EUROPEAN SEARCH REPORT** | Application Number EP 23 21 3715 |

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2022/375540 A1 (CHABON JACOB J [US] ET AL) 24 November 2022 (2022-11-24) <br> * paragraph [0030] * <br> * paragraph [0055] * <br> * paragraph [0154] * <br> * claims 1-8 * <br> * paragraph [0299] * <br> * paragraph [0453] * <br> * paragraph [0476] - paragraph [0477] * <br> * paragraph [0489] - paragraph [0492] * <br> * paragraph [0497] * <br> * paragraph [0499] * <br> * paragraph [0682] * <br> * paragraph [0684] - paragraph [0685] * <br> * paragraph [0695] * <br> * paragraph [0724] * <br> * paragraph [0764] * <br> ----- | 1-15 | INV. <br> G16B20/00 <br> C12Q1/6886 <br> G16B20/20 <br> G16B30/00 <br> G16H50/20 |
| X | US 2021/002728 A1 (LANDAU DAN AVI [US] ET AL) 7 January 2021 (2021-01-07) <br> * paragraph [0007] - paragraph [0013] * <br> * paragraph [0141] - paragraph [0159] * <br> * paragraph [0238] - paragraph [0331] * <br> * paragraph [0345] * <br> * claims 1-4 * <br> ----- | 1-15 | |
| | | | TECHNICAL FIELDS SEARCHED (IPC) |
| X | CN 112 020 563 A (CANCER RESEARCH TECH LTD) 1 December 2020 (2020-12-01) <br> * claims 1,8-9 * <br> * paragraph [0037] - paragraph [0038] * <br> * paragraph [0209] * <br> ----- | 1-15 | G16B <br> C12Q <br> G16H |
| A | US 2019/256924 A1 (VOGELSTEIN BERT [US] ET AL) 22 August 2019 (2019-08-22) <br> * paragraph [0503] * <br> * claim 17 * <br> ----- | 1-15 | |

-/--

The present search report has been drawn up for all claims

| Place of search | Date of completion of the search | Examiner |
|---|---|---|
| Munich | 14 May 2024 | Martínez Cebollada |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt
European
Patent Office
Office européen
des brevets

# EUROPEAN SEARCH REPORT

Application Number

EP 23 21 3715

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2020/248266 A1 (SWANTON ROBERT CHARLES [GB] ET AL) 6 August 2020 (2020-08-06) * paragraph [0139] * | 1-15 | |

TECHNICAL FIELDS
SEARCHED      (IPC)

The present search report has been drawn up for all claims

| Place of search | Date of completion of the search | Examiner |
|---|---|---|
| Munich | 14 May 2024 | Martínez Cebollada |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 23 21 3715

This annex lists the patent family members relating to the patent documents cited in the above-mentioned European search report.
The members are as contained in the European Patent Office EDP file on
The European Patent Office is in no way liable for these particulars which are merely given for the purpose of information.

14-05-2024

| Patent document cited in search report | Publication date | Patent family member(s) | | Publication date |
|---|---|---|---|---|
| US 2022375540 A1 | 24-11-2022 | US | 2022375540 A1 | 24-11-2022 |
| | | US | 2024105281 A1 | 28-03-2024 |
| US 2021002728 A1 | 07-01-2021 | AU | 2019228512 A1 | 03-09-2020 |
| | | CA | 3092352 A1 | 06-09-2019 |
| | | CN | 112602156 A | 02-04-2021 |
| | | EP | 3759238 A1 | 06-01-2021 |
| | | IL | 276893 A | 29-10-2020 |
| | | JP | 2021520004 A | 12-08-2021 |
| | | KR | 20210003094 A | 11-01-2021 |
| | | SG | 11202007871R A | 29-09-2020 |
| | | US | 2021002728 A1 | 07-01-2021 |
| | | US | 2023295738 A1 | 21-09-2023 |
| | | WO | 2019169044 A1 | 06-09-2019 |
| CN 112020563 A | 01-12-2020 | AU | 2019229606 A1 | 15-10-2020 |
| | | CA | 3093092 A1 | 12-09-2019 |
| | | CN | 112020563 A | 01-12-2020 |
| | | EP | 3762512 A1 | 13-01-2021 |
| | | JP | 2021516962 A | 15-07-2021 |
| | | US | 2020402613 A1 | 24-12-2020 |
| | | WO | 2019170773 A1 | 12-09-2019 |
| US 2019256924 A1 | 22-08-2019 | AU | 2018342007 A1 | 27-02-2020 |
| | | BR | 112020002555 A2 | 11-08-2020 |
| | | CA | 3072195 A1 | 04-04-2019 |
| | | CL | 2020000343 A1 | 19-03-2021 |
| | | CN | 111868260 A | 30-10-2020 |
| | | EP | 3665308 A1 | 17-06-2020 |
| | | EP | 3837385 A1 | 23-06-2021 |
| | | IL | 272470 A | 31-03-2020 |
| | | JP | 7232476 B2 | 08-03-2023 |
| | | JP | 2020530290 A | 22-10-2020 |
| | | JP | 2023075090 A | 30-05-2023 |
| | | KR | 20200115450 A | 07-10-2020 |
| | | SG | 11202001010U A | 30-03-2020 |
| | | US | 2019256924 A1 | 22-08-2019 |
| | | US | 2020377956 A1 | 03-12-2020 |
| | | WO | 2019067092 A1 | 04-04-2019 |
| | | WO | 2020150656 A1 | 23-07-2020 |
| US 2020248266 A1 | 06-08-2020 | AU | 2017354900 A1 | 16-05-2019 |
| | | CA | 3041825 A1 | 11-05-2018 |
| | | CN | 110462065 A | 15-11-2019 |
| | | EP | 3535419 A1 | 11-09-2019 |
| | | JP | 7211941 B2 | 24-01-2023 |

EPO FORM P0459

For more details about this annex : see Official Journal of the European Patent Office, No. 12/82

## ANNEX TO THE EUROPEAN SEARCH REPORT
## ON EUROPEAN PATENT APPLICATION NO.

**EP 23 21 3715**

This annex lists the patent family members relating to the patent documents cited in the above-mentioned European search report.
The members are as contained in the European Patent Office EDP file on
The European Patent Office is in no way liable for these particulars which are merely given for the purpose of information.

**14-05-2024**

| Patent document cited in search report | Publication date | Patent family member(s) | | Publication date |
|---|---|---|---|---|
| | | JP 2019533464 A | | 21-11-2019 |
| | | US 2020248266 A1 | | 06-08-2020 |
| | | WO 2018083467 A1 | | 11-05-2018 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2022125997 A1